# EUROPEAN PATENT APPLICATION

(11) **EP 2 277 525 A1**
(43) Date of publication of application: **26.01.2011**
(21) Application number: 08748612.2
(22) Date of filing: 19.05.2008
(51) Int. Cl.: A61K 36/11, A61K 31/047, A61P 3/10

(54) **A METHOD FOR PREPARING EXTRACTIVE CONTAINING SEQUOYITOL FROM NEPHROLEPIS FAMILY AND APPLICATION**

(71) Applicant: Guangzhou Welman New Drug R&D Co,. Ltd., Shuiyin Road No. 56, Yuexiu District Guangzhou Guangdong 510075 (CN)
(72) Inventor: SUN, Tianyu, Guangdong 510075 (CN); WANG, Yanli, Guangdong 510075 (CN); WANG, Yinyin, Guangdong 510075 (CN); LU, You, Guangdong 510075 (CN); WANG, Ting, Guangdong 510075 (CN)
(74) Representative: Laufhütte, Dieter
(86) International application number: PCT/CN2008/071006
(87) International publication number: WO 2009/140814

(57) **Abstract**

A method for obtaining a sequoyitol-containing extract from a plant of genus *Nephrolepis,* comprising extracting with a solvent, recovering the solvent to give an extractum, subjecting the extractum to two-phase extraction and column chromatography, collecting the fractions containing sequoyitol, concentrating, filtration and drying. The main active ingredient of the extract obtained is sequoyitol, which can be used for treating diabetes and its complications.

## Description

### Technical Field

The present invention relates to a method for obtaining a sequoyitol-containing extract and the use of the extract. In particular, the present invention relates to a method for obtaining a sequoyitol-containing extract from a plant of genus *Nephrolepis,* the sequoyitol-containing extract obtained by the method and the use of the extract.

### Background of the Invention

In recent years, along with the rapid development of national economy and the unceasing enhancement of people's living level in China, the incidences of some diseases that affect people's health alter accordingly. The increasingly incidence of diabetes and lowering ages of patients is especially prominent.

Diabetes is a metabolic disease characterized by high levels of blood glucose and severely affects people's health. It may cause the vascular pathology of the heart, brain, kidney, lower limbs and the like, and may lead to severe consequences, such as hypertension, heart disease, diabetic nephropathy, numbness of lower limb and gangrene and the like. The pathogenic mechanism of diabetes has not been well understood by now, and the majority of therapies of diabetes are focused on lowering the high level of blood glucose and the prophylaxis of complications. Since the 1950's, the drugs for treating diabetes are mainly divided into 8 groups: insulin, sulfonylureas, biguanides, alpha-glucosidase inhibitors, insulin release enhancers, insulin sensitizers, aldose reductase inhibitors and gluconeogenesis inhibitors. The above drugs have inevitable disadvantage of strong side effects, damage to other organs and strong drug resistance. Thus, there is a great need for a drug from natural plants that can be used to treat diabetes with high safety and efficiency as well as low cost.

Inositols, such as myo-inositol and D-chiro-inositol, play an important role in the signaling of insulin's effects. It is found in some studies that a certain concentration of D-chiro-inositol is detected in normal blood and urine, however, D-chiro-inositol in blood of patients with type II diabetes is undetected and D-chiro-inositol in diabetic urine is several times higher than that in normal urine. Thus, it suggests that D-chiro-inositol in these patients flows away rapidly due to metabolic disorder and ultimately results in the blockade of signal transmission of insulin action. The results from animal experiments indicate that inositols may promote the function of insulin and lower the levels of blood glucose, blood insulin and blood triglycerides and the like. Moreover, it also has a protective effect on diabetic retinal pericapillary cells. The methyl derivatives of inositols also have the effect of decreasing blood glucose.

The present invention provides sequoyitol extracted from plants, which is a myo-inositol derivative. Pharmacological experiments show that it has prophylactic and therapeutic effects on diabetes.

Chinese patent application for invention No. 02144302.5 by Liang Jingyu et al. discloses that the sequoyitol isolated from *Taxus chinensis* is an antidiabetes drug with very low toxicity that remarkably decreases high glycemia in diabetes models, inhibits the breakdown of hepatic glycogen and the absorption of glucose. However, *Taxus chinensis* is under plant protection and there are no abundant sources available. Accordingly, it is not applicable to study and develop in large scale. In order to find the alternative source for *Taxus chinensis,* we use a plant of genus Nephrolepis as the source of sequoyitol. Although it is reported that the plants of genus Nephrolepis contain sequoyitol, the process used is only aimed at its identification, and the content of sequoyitol obtained by extract process is very low, so the process cannot meet the requirement of industrial production in terms of the cost of extract and the route of process.

### Summary of the Invention

An object of the present invention is to provide a method for obtaining a sequoyitol-containing extract from a plant of genus *Nephrolepis.*

Another object of the present invention is to provide a sequoyitol-containing extract prepared by the above-mentioned method.

A further object of the present invention is to provide the use of the sequoyitol-containing extract or pharmaceutically acceptable salts thereof for the preparation of a medicament for treating diabetes and its complications.

The present invention relates to a method for obtaining a sequoyitol-containing extract from a plant of genus *Nephrolepis,* comprising the steps of extracting with a solvent, recovering the solvent to give an extractum, subjecting the extractum to two-phase extraction and column chromatography, collecting the fractions containing sequoyitol, concentrating, filtrating and drying.

Preferably, the plant of genus Nephrolepis is selected from the group consisting of *Nehprolepis auriculata(L.)Trimen, N. biserrata, N. duffii Moore, N. falcata(Cav)C.Chr., N. delicatula(Decne.) Pichi-Serm., N. biserrata(Sw.) Schott., N. biserrata var. auriculata Ching, N. exaltata(Sw.)Schott., N. hirsutula(Forst.)Presl, N. cordifolia, N. exaltata cv. Bostoniensis,* and any combination thereof.

Preferably, the solvent used in the extraction step is selected from the group consisting of ethanol, methanol, acetone, water and any combinations thereof. Preferably, the temperature of the extraction is 0 °C to the reflux temperature of the solvent, preferably room temperature to the reflux temperature of the solvent, more preferably the reflux temperature of the solvent. Preferably, the weight to volume ratio of the solvent and the plant of genus *Nephrolepis* is 1:1 to 20, preferably 1:2 to 10, more preferably 1:3 to 5. Preferably, the extraction is performed 2∼5 times.

Preferably, the solvents used for the two-phase extraction are a water-immiscible organic solvent and water. Preferably, the water-immiscible organic solvent is selected from the group consisting of ethyl acetate, chloroform, dichloromethane, ethyl ether, petroleum ether and n-butanol. Preferably, the ratio of the water-immiscible organic solvent to water is 1:1 to 10 (v/v).

Preferably, the column chromatography is macroporus resin column chromatography, sephadex column chromatography, modified sephadex column chromatography, cellulose column chromatography, activated charcoal column chromatography or ion-exchange resin column chromatography.

Preferably, a purification step is performed after the filtration and prior to the drying. Preferably, the purification step is column chromatography.

The present invention further provides the extract prepared from the above-mentioned method for obtaining a sequoyitol-containing extract.

The present invention further provides the use of the extract prepared by the above method or pharmaceutically acceptable salts thereof for the preparation of medicaments for treating diabetes and its complications. Preferably, the pharmaceutically acceptable salts are selected from the following salts of sequoyitol: citrate, tartrate, acetate, lactate, sodium salt, potassium salt, calcium salt, ammonium salt, magnesium salt, ferric salt, zinc salt, aluminum salt, and any combination thereof. Preferably, the complication is selected from ocular disorders, kidney disorders, neuropathy, coronary heart disease, cerebrovascular disorders and peripheral vascular disorders.

The present method for obtaining a sequoyitol-containing extract from a plant of genus *Nephrolepis* puts forward an optimized extraction process according to the inherit characteristic of plants of genus *Nephrolepis,* and presents a strategy for industrial production. The sequoyitol-containing extract obtained by the method shows an antidiabetic activity and low toxicity. The major active ingredient of the extract is sequoyitol, the content of which is between 10.00% and 99.99%. Animal experiments show that the extract could remarkably decrease the level of blood glucose.

### Breif Description of the Drawings

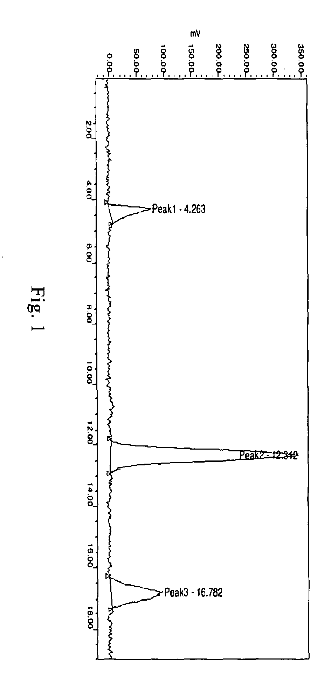

Figure. 1 shows the retention time of sequoyitol in Example 9.

### Detailed Description of the Preferred Embodiment

A detailed description of the technical solution of the present invention is as follows.

The present invention provides a method for extracting and isolating a sequoyitol-containing extract with an antidiabetic activity and low toxicity from a plant of genus *Nephrolepis* including Nephrolepidaceae. Preferably, the plant of genus Nephrolepis is selected from the group consisting of *Nehprolepis auriculata(L.)Trimen, N. biserrata, N. duffi Moore, N. falcata(Cav)C. Chr., N. delicatula(Decne.) Pichi-Serm., N. biserrata(Sw.) Schott., N. biserrata var. auriculata Ching, N. exaltata(Sw.)Schott., N. hirsutula(Forst.)Presl, N. cordifolia and N. exalatata cv. Bostoniensis* etc.

The present invention relates to a method for obtaining a sequoyitol-containing extract from a plant of genus *Nephrolepis,* comprising the steps of extracting with a solvent, recovering the solvents to give an extractum, subjecting the extractum to two-phase extraction and column chromatography, collecting the fractions containing sequoyitol, concentration, filtration and drying. The sequoyitol-containing extract obtained by the method is usually a crystalline powder, in which the content of sequoyitol may be up to more than 60%.

The sequoyitol-containing extract obtained by the above method may be used for the preparation of medicaments and pharmaceutical compositions for treating diabetes and its complications. The sequoyitol-containing extract may be used to prepare injections, capsules, tablets, granules, dragees, solutions etc, or controlled release preparations or targeting preparations of the above dosage forms, in combination with one or more auxiliaries and/or excipients.

Sequoyitol, as well as its pharmaceutically acceptable salts obtained by reacting sequoyitol with corresponding acids or bases, may be used in the preparation of the preparations of the invention.

The sequoyitol-containing extract obtained by the present method may be used for the preparation of medicaments for treating diabetes, may markedly decrease diabetic hyperglycemia, inhibit the breakdown of hepatic glycogen and the absorption of glucose, decrease the level of blood fat, improve the metabolism of free radicals and protect islet beta-cells, while with low toxicity. The sequoyitol-containing extract obtained by the present method may also be used for the prevention and treatment of diabetes and its complications, or other diseases associated with glycometabolism disorder, type II diabetes and its complications, and for the improvement of the metabolism of free radicals.

In particular, the present method for obtaining a sequoyitol-containing extract from a plant of genus *Nephrolepis* typically comprises the following steps. The parts (such as root, stem and leave etc.) of a plant of genus *Nephrolepis* are dried in the air and smashed, and then extracted with a solvent such as ethanol, methanol, acetone, or the hydrate or mixtures thereof etc. at a temperature in the range of 0°C to the reflux temperature, preferably room temperature to the reflux temperature, more preferably the reflux temperature. Preferably, the amount of the solvent used is 1:1 to 20 (w/v), preferably 1:2 to 10 (w/v), more preferably 1:3 to 5 (w/v). Preferably, the extraction is performed 2-5 times. After concentration under reduced pressure an extractum is obtained, then the extractum is subjected to two-phase extraction with water-immiscible organic solvents (such as ethyl acetate, chloroform, dichloromethane, ethyl ether, petroleum ether, etc.) and water, in which the extraction agent is preferably organic solvent/water in the ratio of 1:1 to 10 (v/v). The organic layer is removed. The aqueous layer is pooled, filtrated, and subjected to column chromatography. The column chromatography may be macroporus resin column chromatography (for example, D101, MN-200 (PUROLITE) and the like), sephadex G column chromatography or modified sephadex column chromatography (for example, sephadex-LH-20 and the like), cellulose column chromatography, activated charcoal column chromatography or ion-exchange resin column chromatography. The elution is performed with corresponding eluent while the eluate is detected. The fractions containing sequoyitol are collected, concentrated under reduced pressure, and purified by column chromatography. The column chromatography may be macroporus resin column chromatography (for example, D101, MN-200 (PUROLITE) and the like), sephadex G or modified sephadex column chromatography (for example, sephadex-LH-20 and the like), cellulose column chromatography, activated charcoal column chromatography or ion-exchange resin column chromatography. The fractions containing sequoyitol are pooled, concentrated under reduced pressure, allowed to set, and filtrated to give a solid, and then recrystallized from ethanol, methanol, acetone, methyl ethyl ketone, or hydrates or mixtures thereof to give a dried powder of plant extracts. The final product is crystalline powder in which the major active ingredient is sequoyitol with purity between 10.00% and 99.99%.

The following method may be used for determining the eluate in the column chromatography of the inventive method.

Thin layer chromatography: F254 thin layer plate is used. 5µl of the test solution or a control solution (aqueous solution of sequoyitol) are loaded on the thin layer plate respectively, then developed with a developing agent containing water, ethyl acetate and isopropanol (6:11:83, in volume), visualized by potassium permanganate-sodium hydroxide. The main spot of the test solution with an Rf value equaling to the control solution indicates a positive reaction.

The content of sequoyitol, the major active ingredient, of the extract of the present invention may be determined by the following High Performance Liquid Chromatography (HPLC):

Assay 1: About 5mg of extract is precisely weighed and charged into a 10 ml volumetric flask. 0.25 ml of diluted sulfuric acid-acetic anhydride (1:50 (v/v)) is added, heated in water-bath for 30 minutes, then cooled to room temperature. 5 ml of methanol is added, evenly mixed. Then water is added to the scale, and evenly mixed to give the test solution. The conditions of HPLC are as follows:
Column: C₁₈ column, 5µl, 4.6x250mm,
Detection wavelength: 220nm,
Sample volume: 20µl,
Mobile phase: methanol/water (50:50 (v/v)), filtrated by 0.45µm organic film filter and degased before use,
Flow rate: 1.0ml/min.

Assay 2: About 5mg of extract is precisely weighed and charged into a 10 ml volumetric flask, then dissolved in water to the desired volume, and evenly mixed. The conditions of HPLC are as follows:
Column: amino column, 5µl, 4.6x250mm,
Detector: Evaporative Light Scattering Detector (ELSD),
Temperature of the drift tube: 40 °C,
Pressure: 3.5 bar,
Sample volume: 20 µl,
Mobile phase: acetonitrile/water (75:25 (v/v)), filtrated by 0.45µm organic film filter and degased before use,
Flow rate: 0.8ml/min.

In clinic, the extract obtained from genus *Nephrolepis* may be used for the preparation of various medicaments or health care products etc.

One or more conventional excipients may be comprised in the dosage forms of capsule, tablet, granule, dregee etc. In particular, the excipients may be as follows:
Filler and solubilizer: starch, microcrystalline cellulose, etc.,
Binder: carboxymethylcellulose, polyvinyl pyrrolidone, etc.,
Wetting agent: glycerol, etc.,
Disintegrating agent: calcium carbonate, etc.,
Absorption agent: kaoline, etc.,
Lubricant: talc powder, etc.

The pharmaceutical excipients may be in the form of solid, semi-solid, liquid or other forms. Various types of auxiliaries can be used in formulation.

Solutions can comprise conventional solvents, solubilizers, emulsifiers, preservatives, etc, such as water, ethanol, glycerol, polyethylene glycol, benzyl benzoate, etc.

The dosage forms, such as capsule, tablet, granule, dregee and solution, may be prepared by known methods. The plant extracts may be mixed with one or more excipients to prepare the dosage forms.

Preparations containing traditional Chinese medicinal extracts for treating diabetes may be prepared according to the present invention, thus new alternatives for the treatment of diabetes are provided. In addition, the plants of genus *Nephrolepis* are abundant and well available traditional Chinese medicine in Southern China. In some regions they are planted in large scale and used as food or raw medicine material. Moreover, its production process is simple and low-cost. A natural medicine which has significant therapeutic effect on diabetes is thereby provided.

The present invention is further described in combination with the following examples.

### Example 1: the preparation of plant extracts with lowest purity

100 kg of crude powder of *Nephrolepis auriculata(L.)Trimen* was extracted three times under refluxing in 3 volumes of 80% ethanol. The resulting extract solution was combined, concentrated under reduced pressure, and extracted by a two-phase system of ethyl acetate and water (1:1 (v/v)). The aqueous layers were pooled, filtrated, and subjected to macroporus resin column chromatography (D101, made in China). Elution was performed with gradients of distilled water and aqueous ethanol. The fractions containing sequoyitol were collected, concentrated under reduced pressure, and dried to give a plant extract (yellowish powder), in which the content of sequoyitol was 10.2%.

### Example 2: the preparation of plant extract with low purity-1

50 kg of crude powder of *Nephrolepis auriculata(L.)Trimen* was extracted three times under refluxing in 3 volumes of 90% ethanol at 60 °C. The resulting extract solution was combined, concentrated to dryness under reduced pressure, and extracted with a two-phase system of dichloromethane and water (1:1 (v/v)). The aqueous layers were combined, filtrated, and subjected to macroporus resin column chromatography (D101, made in China). Elution was performed with gradients of distilled water and aqueous ethanol (ethanol: 5%∼20%). The eluates were detected with detection reagents respectively. The eluates exhibiting positive reactions were pooled, concentrated under reduced pressure, and dried at 70 °C to give a plant extract (yellowish powder), in which the content of sequoyitol was 28.9%.

### Example 3: the preparation of plant extract with low purity-2

60 kg of crude powder of *Nephrolepis auriculata(L.)Trimen* was extracted three times under refluxing in 3 volumes of 90% methanol at 50 °C. The resulting extract solution was combined, concentrated to dryness under reduced pressure, and extracted with a two-phase system of ethyl acetate and water (1:1 (v/v)). The aqueous layers were combined, filtrated, and subjected to macroporus resin column chromatography (MN-200, made in China). Elution was performed with gradients of distilled water and aqueous ethanol (ethanol: 5%∼ 20%). The eluates were detected with detection reagents respectively. The eluates exhibiting positive reactions were pooled, concentrated under reduced pressure, and dried at 70 °C to give a plant extract (yellowish powder), in which the content of sequoyitol was 29.5%.

### Example 4: the preparation of plant extract of low purity-3

70 kg of crude powder of *Nephrolepis auriculata(L.)Trimen* was extracted three times under refluxing in 3 volumes of 70% acetone at 45 °C. The resulting extract solution was combined, concentrated to dryness under reduced pressure, and extracted with a two-phase system of chloroform and water (1:1 (v/v)). The aqueous layers were combined, filtrated, and subjected to activated charcoal column chromatography. Elution was performed with gradients of distilled water and aqueous ethanol (ethanol: 5%∼25%). The eluates were detected with detection reagents respectively. The eluates exhibiting positive reactions were pooled, concentrated under reduced pressure, and dried at 70 °C to give a plant extract (yellowish powder), in which the content of sequoyitol was 30.2%.

### Example 5: the preparation of plant extract with moderate purity-1

The plant extract with lowest purity was dissolved in water, and subjected to cellulose column chromatography. Elution was performed with gradients of distilled water and aqueous ethanol (ethanol: 5%∼ 20%). The eluates were detected with detection reagents respectively. The eluates exhibiting positive reactions were pooled, concentrated under reduced pressure, recrystallized in ethanol, and filtrated to give a crystalline powder. The resulting crystalline powder was vacuum dried, and dried at 70 °C to give a plant extract (yellowish powder), in which the content of sequoyitol was 78%, as determined by HPLC.

### Example 6: the preparation of plant extract with moderate purity-2

The plant extract with lowest purity was dissolved in water, and subjected to ion exchange column chromatography. Elution was performed with gradients of distilled water and aqueous ethanol (ethanol: 5% ∼ 20%). The eluates were detected with detection reagents respectively. The eluates exhibiting positive reactions were pooled, concentrated to dryness under reduced pressure, recrystallized in methanol, and filtrated to give a crystalline powder. The resulting crystalline powder was vacuum dried, and dried at 70 °C to give a plant extract (yellowish powder), in which the content of sequoyitol was 75%, as determined by HPLC.

### Example 7: the preparation of plant extract with moderate purity-3

The plant extract with lowest purity was dissolved in water, and subjected to gel column chromatography. Elution was performed with gradients of distilled water and aqueous ethanol (ethanol: 5%∼20%). The eluates were detected with detection reagents respectively. The eluates exhibiting positive reactions were pooled, concentrated to dryness under reduced pressure, recrystallized in acetone, and filtrated to give a crystalline powder. The resulting crystalline powder was vacuum dried, and dried at 70 °C to give a plant extract (yellowish powder), in which the content of sequoyitol was 68%, as determined by HPLC.

### Example 8: the preparation of plant extract with high purity

The plant extract with moderate purity was recrystallized repeatedly in methanol and the like, filtrated to give a crystalline powder. The resulting crystalline powder was vacuum dried, and dried at 70 °C to give a plant extract with high purity of sequoyitol. After repeated recrystallization, the purity of sequoyitol was as high as more than 99%.

### Example 9: the determination of the content of the sequoyitol extract with high purity

HPLC was used. About 5mg of the extract of high purity was precisely weighed and charged into a 10 ml volumetric flask, then dissolved in water to the desired volume, and mixed homogeneously. The conditions of HPLC were as follows:
Column: amino column, 5µl, 4.6x250mm,
Detector: Evaporative Light Scattering Detector (ELSD),
Temperature of the drift tube: 40 °C,
Pressure: 3.5 bar,
Sample volume: 20 µl,
Mobile phase: acetonitrile/water (75:25 (v/v)), filtrated by 0.45µm organic film filter and degased before use,
Flow rate: 1.0 ml/min.

The spectra are shown in figure 1, in which the retention time of sequoyitol is 12.312 min.

### Example 10: the preparation of capsules

Formula: each capsule weighing 300mg, containing 30mg of plant extract.

| | |
|---|---|
| microcrystalline cellulose | 270 mg |
| extract of plant | 30 mg |
| | 1000 caps |

The excipient was placed in a grinder, then the plant extract was added and mixed by grinding for 10∼30 min to uniform. The homogeneously blended mixture was encapsulated into capsules of size #1. The load of each capsule was 300mg, in which the amount of the plant extract was 30mg.

### Example 11: the stability study on the capsules

A 9-month stability test was carried out for 3 batches of capsules, including examinations on the appearance, identification, content and the like.

Identification (1): The contents of 20 capsules were collected. An appropriate amount of the powder was taken and dissolved in water, and filtrated. 5ml of the filtrate was placed into a tube. After 2ml of sodium periodate solution (0.01mol/L) and 2 drops of concentrated nitric acid were added, silver nitrate solution was added dropwise, and a white precipitate produced. The results for 3 batches of capsules are shown in Table 1.

Identification (2): 10ml of water was added to an appropriate amount of the contents (corresponding to 0.2g of the capsule), shaken up and filtrated. To 2ml of the filtrate, 1ml of basic lead acetate solution was added, mixed homogeneously. The mixture was heated in water bath for 5 min, and a semitransparent jelly was obtained. The results for 3 batches of capsules are shown in Table 1.

**Table 1. The stability of 3 batches of capsules**

| Time (months) | Batch Number | Appearance (colour) | Disintegration time(min) | Identification ( 1 ) | Identification ( 2 ) | Content ( % ) |
|---|---|---|---|---|---|---|
| 0 | 051116 | Off white | ≤ 10 | eligible | eligible | 72. 8 |
| | 051117 | Off white | ≤10 | eligible | eligible | 69. 5 |
| | 051118 | Off white | ≤ 10 | eligible | eligible | 70. 6 |
| 3 | 051116 | Off white | ≤ 10 | eligible | eligible | 71. 0 |
| | 051117 | Off white | ≤ 10 | eligible | eligible | 70. 5 |
| | 051118 | Off white | ≤10 | eligible | eligible | 68.9 |
| 6 | 051116 | Off white | ≤ 10 | eligible | eligible | 72. 0 |
| | 051117 | Off white | ≤ 10 | eligible | eligible | 68. 7 |
| | 051118 | Off white | ≤ 10 | eligible | eligible | 69. 1 |
| 9 | 051116 | Off white | ≤ 10 | eligible | eligible | 71.5 |
| | 051117 | Off white | ≤ 10 | eligible | eligible | 70.5 |
| | 051118 | Off white | ≤ 10 | eligible | eligible | 69. 3 |

### Example 12: the preparation of tablets

25g of plant extract, 49g of microcrystalline cellulose and 1g of magnesium stearate were well mixed. The resulting mixture was pressed into tablets using single punch tablet press. The tablets were 6 mm in diameter and 300 mg in weight, and each contained 100mg of plant extract.

### Example 13: the preparation of granules

20g of plant extract and 180g of corn starch were well mixed. An appropriate amount of 60% ethanol was added to obtain a dough. Then the dough was screened through a 12-mesh sieve to granulate. Granules were obtained after drying. Each gram of granule contained 100mg of plant extract.

### Example 14: the preparation of injections

The refined sequoyitol extract (the purity was 98.00%∼99.99%) was used as the starting material of injections.

Sequoyitol are well soluble in water. Sequoyitol was dissolved in water and sterilized by filtration. Solutions for injection were prepared according to the operation procedure of solutions for injection. Powder for injection was prepared according to the operation procedure of lyophilized powder for injection.

Lyophilized powder for injection was prepared from the refined sequoyitol for injection, according to the operation procedure of lyophilized powder for injection. The sequoyitol for injection was dissolved in water for injection. 20% low molecule dextran solution (free of pyrogen) was added. The resulting solution was well mixed. The final concentrations of sequoyitol and dextran were 0.1 g/ml and 0.05g/ml respectively. Pyrogen was removed by activated charcoal. The resulted solution was sterilized by a 0.22µm micro filter membrane, and filled into pretreated vials in a clean zone of class 100. Each vial contains 1ml of solution. In a freeze dryer, the vials were freezed at -40 °C for 2 hours, and then sublimated at -25 °C under vacuum of 1.33 Pa. After 90% of free water was removed, the vials were dried by heating. The temperature was kept no higher than 35 °C over the course of lyophylization. The vials were sealed with stoppers in the freeze dryer.

### Example 15: the preparation of dispersible tablets

60g of dry sequoyitol extract, 20g of microcrystalline cellulose, 10g of cross-linked polyvinylpyrrolidone and 10g of low-substituted hydroxyproxyl cellulose were well mixed. 5% polyvinylpyrrolidone K30 in ethanol was added dropwise to obtain a dough. The dough was screened through a 30-mesh sieve to granulate. The wet granules were dried at 60 °C, then screened through a 40-mesh sieve. 10g of cross-linked polyvinylpyrrolidone, 10g of low-substituted hydroxyproxyl cellulose and 5g of magnesium stearate were added and well mixed. The resulting mixture was compressed into 1000 tablets.

Determination: (1) Determination of the uniformity of dispersion: 2 dispersible tablets of sequoyitol extract were placed in 100ml water and shaken. They disintegrated completely in 180s at 20°C±1°C and passed through a 2# sieve. (2) The determination of dissolution: the dissolution was determined using artificial gastric fluid (0.1 mol/L hydrochloric acid). The determination was carried out according to the second method (paddle) of the determination of dissolution appendix to the Pharmacopoeia of the People's Republic of China (Part II), at a paddle speed of 100 r/min. The dissolution temperature was 37 °C, and the volume of the dissolution medium was 900ml. Dispersible tablets of sequoyitol extract were added into the above dissolution medium under said conditions. 5 ml of aliquots were sampled at 2, 4, 6, 8, 10, 15, 20, 30, 45 and 60 minutes (and supplemented with the same amount of medium), and filtered with 45µm microporous filter membrane. The dissolution was determined, and then relative cumulative dissolution percentages were calculated. The results are shown in Table 2.

**Table 2. The dissolution of dispersible tablets of sequoyitol extract**

| Time (min) | 2 | 4 | 6 | 8 | 10 | 15 | 20 | 30 | 45 | 60 |
|---|---|---|---|---|---|---|---|---|---|---|
| Dissolution (%) | 73.24 | 81.34 | 85.54 | 91.36 | 92. 42 | 94. 19 | 95. 29 | 96. 26 | 96. 42 | 97. 28 |

### Example 16: the preparation of effervescent tablets

Effervescent tablets of sequoyitol extract were prepared according to the following formula: 60g of sequoyitol extract, 4.2g of tartaric acid, 17.5g of sodium bicarbonate, 1.2g of Tween-80, 3.2g of polyvinylpyrrolidone(PVP), 1.6g of low-substituted hydroxyproxyl cellulose (LHPC), and 0.64g of magnesium stearate were well mixed and compressed into 1000 tablets. Each tablet contains 60mg of sequoyitol extract.

Examination: 10 test tubes with stopper and graduation (internal diameter: 1.5cm, capacity: 25ml) were filled with precisely 2ml of water respectively, then placed in water bath at 37°C±1°C. 5 minutes later, to the test tube was added one test tablet, and then was sealed with the stopper. The volume of greatest effervescent amount was observed in a period of 20 minutes. The mean effervescent volume was 4.6 ml, with one tablet being less than 3ml.

### Example 17: the preparation of syrup

600g of sucrose was placed in a 1000 ml beaker. 800ml distilled water was added and heated to dissolve the sucrose. 20g of sequoyitol extract was added and dissolved by stirring. The solution was cooled to room temperature and filtrated. The filtrate was collected, and distilled water was added to 1000ml. After well mixed, the filtrate was filled into 102ml ampoules, and sterilized at 100°C for 30 minutes.

The pharmacodynamic and toxicological tests were performed on the plant extract prepared according to example 1.

### Pharmacodynamic test

The effect of the plant extract on the elevated level of blood glucose induced by alloxan in mice

### Method

80 male mice weighed 22 ∼ 25g were used. 20 mice were randomly taken as the blank group. Others were injected intravenously with alloxan via caudal vein at a dose of 65mg/kg body weight, and then modeled according to established methods. The mice were divided into 3 groups according to their level of blood glucose, in which 2 groups were administered via gavage with 60mg/kg of the *Nephrolepis auriculata* extract, 75mg/kg of phenformin (positive control) respectively for consecutive 7 days, in a volume of 0.1ml/10g body weight. The same volume of distilled water was administered to the blank group and the control group. Blood glucose was determined using glucose oxidase method.

### Results

The blood glucose levels of the control group were remarkably elevated (by 323.7mg/dl) as compared to the blank group. In comparison to the control group, the hyperglycemia induced by alloxan was remarkably decreased in the 75mg/kg phenformin group (by 152.8 mg/dl) and the *Nephrolepis auriculata* extract group (by 110.6 mg/dl).
The effect of the plant extract on elevated blood glucose level induced by glucose in mice

### Method

120 male mice weighed 22 ∼ 25g were randomly divided into 6 groups, in which 4 groups were administered via gavage with 60mg/kg of the *Nephrolepis auriculata* extract (the content of sequoyitol was 65%) for consecutive 7 days in a volume of 0.1 ml/10g body weight. The same volume of distilled water was administered to the blank group and control group. The blank group was injected intraperitoneally with physiological saline, while the other groups were injected intraperitoneally with 2g/kg of glucose in the same volume. Blood was collected from the posterior orbital venous plexus at 30, 60, 90 and 120 minutes after injection and the serum was isolated. Blood glucose was determined using glucose oxidase method.

### Results

The blood glucose levels of mice in the control group were remarkably elevated at 30, 60, 90 and 120 minutes after the injection of glucose, as compared to the blank group. The hyperglycemia induced by the injection of glucose was remarkably decreased by the *Nephrolepis auriculata* extract at 30, 60, 90 and 120 minutes after the injection of glucose, as compared to the control group. At 90 minutes after the injection, the hyperglycemia was decreased by 15.5mg/dl in the *Nephrolepis auriculata* extract.

### Acute toxicity test

Method: 40 healthy mice (20 male and 20 female) were administered via gavage once with 800 mg/kg of *Nephrolepis auriculata* extract and monitored. 7 days later, the mice were sacrificed, and the organs were examined visually. Results: No fatality was observed. All the mice were healthy, with their skins and hair lustrous and their eyes bright, and showed good motility. After 7 days, all the mice were sacrificed, the major organs were examined visually and no abnormity was found.

The foregoing descriptions merely describe some preferred embodiments of the present invention. It should be noted that various improvements and modifications may be made by those skilled in the art, without departing from the principle of the invention. Thus these improvements and modifications still fall within the scope of the present invention.

## Claims

1. A method for obtaining a sequoyitol-containing extract from a plant of genus *Nephrolepis,* comprising the steps of extracting with a solvent, recovering the solvent to give an extractum, subjecting the extractum to two-phase extraction and column chromatography, collecting the fractions containing sequoyitol, concentrating, filtrating and drying.

2. The method for obtaining a sequoyitol-containing extract according to claim 1, wherein the plant of genus *Nephrolepis* is selected from the group consisting of *Nehprolepis auriculata(L.)Trimen, N. biserrata, N. duffii Moore, N. falcata(Cav.)C.Chr., N. delicatula(Decne.) Pichi-Serm., N. biserrata(Sw) Schott., N. biserrata var. auriculata Ching, N. exaltata(Sw.)Schott., N. hirsutula(Forst.)Presl, N. cordifolia, N. exalatata cv. Bostoniensis,* and any combination thereof.

3. The method for obtaining a sequoyitol-containing extract according to claim 1, wherein the solvent is selected from the group consisting of ethanol, methanol, acetone, water, and any combination thereof.

4. The method for obtaining a sequoyitol-containing extract according to claim 3, wherein the temperature of the extraction step is between 0 °C and the reflux temperature of the solvent, preferably between room temperature and the reflux temperature of the solvent, more preferably the reflux temperature of the solvent.

5. The method for obtaining a sequoyitol-containing extract according to claim 4, wherein the weight to volume ratio of the solvent and the plant of genus Nephrolepis is 1:1 to 20, preferably 1:2 to 10, more preferably 1:3 to 5.

6. The method for obtaining a sequoyitol-containing extract according to claim 1, wherein the extraction is performed 2∼5 times.

7. The method for obtaining a sequoyitol-containing extract according to claim 1, wherein the solvent used in the two-phase extraction is a water-immiscible organic solvent and water.

8. The method for obtaining a sequoyitol-containing extract according to claim 7, wherein the water-immiscible organic solvent is selected from the group consisting of ethyl acetate, chloroform, dichloromethane, ethyl ether, petroleum ether and n-butanol.

9. The method for obtaining a sequoyitol-containing extract according to claim 7, wherein the volume ratio of the water-immiscible organic solvent to water is 1:1 to 10.

10. The method for obtaining a sequoyitol-containing extract according to claim 1, wherein the column chromatography is macroporus resin column chromatography, sephadex column chromatography, modified sephadex column chromatography, cellulose column chromatography, activated charcoal column chromatography or ion-exchange resin column chromatography.

11. The method for obtaining a sequoyitol-containing extract according to claim 1, wherein a purification step is performed after filtrating and before drying.

12. The method for obtaining a sequoyitol-containing extract according to claim 11, wherein the purification is column chromatography.

13. The extract obtainable by the method for obtaining a sequoyitol-containing extract according to any one of claims 1.

14. Use of the extract according to claim 13 or pharmaceutically acceptable salts thereof for the preparation of a medicament for treating diabetes and its complications.

15. The use according to claim 14, wherein the pharmaceutically acceptable salts are selected from the following salts of sequoyitol: citrate, tartrate, acetate, lactate, sodium salt, potassium salt, calcium salt, ammonium salt, magnesium salt, ferric salt, zinc salt, aluminum salt, and any combination thereof.

16. The use according to claim 14, wherein the complication is selected from ocular disorders, kidney disorders, neuropathy, coronary heart disease, cerebrovascular disorders and peripheral vascular disorders.
